# EUROPEAN PATENT SPECIFICATION

(11) **EP 3 433 380 B1**
(45) Date of publication and mention of the grant of the patent: **08.06.2022**
(21) Application number: 17770994.6
(22) Date of filing: 21.03.2017
(51) Int. Cl.: C12Q 1/6883, G01N 33/94

(54) **DRUGS, PHARMACOGENOMICS AND BIOMARKERS FOR ACTIVE LONGEVITY**
ARZNEIMITTEL, PHARMAKOGENOMIK UND BIOMARKER FÜR EINE AKTIVE LANGLEBIGKEIT
MÉDICAMENTS, PHARMACOGÉNOMIQUES ET BIOMARQUEURS DESTINÉS À UNE LONGÉVITÉ ACTIVE

(30) Priority: 21.03.2016 US 201662310942 P
(43) Date of publication of application: 30.01.2019
(73) Proprietor: Indiana University Research & Technology Corporation, Indianapolis, IN 46202 (US); The Scripps Research Institute, La Jolla, CA 92037 (US); The United States Of America As Represented By The Department Of Veterans Affairs, Washington, DC 20420 (US)
(72) Inventor: NICULESCU, Alexander B., Indianapolis, Indiana 46202 (US); PETRASCHECK, Michael, La Jolla, California 92037 (US)
(74) Representative: V.O.
(86) International application number: PCT/US2017/023422
(87) International publication number: WO 2017/165423

(56) References cited:
- WO-A2-2006/138696
- WO-A2-2009/026116
- US-A1- 2008 057 504
- US-A1- 2009 083 868
- US-A1- 2009 092 551
- US-A1- 2010 120 046
- US-A1- 2010 173 024
- US-A1- 2011 207 128
- US-A1- 2011 207 128
- US-A1- 2014 243 211
- US-A1- 2014 274 764
- SANJANA SOOD ET AL: "A novel multi-tissue RNA diagnostic of healthy ageing relates to cognitive health status", GENOME BIOLOGY, vol. 16, no. 1, 7 September 2015 (2015-09-07), XP055618124, DOI: 10.1186/s13059-015-0750-x
- GLEI ET AL.: 'Beyond Self-Reports: Changes in Biomarkers as Predictors of Mortality' POPULATION AND DEVELOPMENT REVIEW vol. 40, no. 2, 01 June 2014, pages 331 - 360, XP055424989

## Description

### BACKGROUND OF THE DISCLOSURE

The present disclosure relates generally to genes and biological pathways involved in mood, stress, and life expectancy. Some of these genes may represent a life switch between suicide and longevity. More particularly, the present disclosure relates to methods for identifying compounds involved in the modulation of longevity by mood and stress, in particular drugs that modulate the life switch. The present disclosure further relates to methods for increasing longevity in a subject in general, and modulating the life switch in particular in a subject with a psychiatric disorder. In one embodiment, the methods utilize drugs that modulate these longevity genes, and the life switch. The present disclosure also relates to methods for determining a biological age score in a subject in general.

The merits of longevity and the perils of aging are the subject of active debate at a societal level, and of concerted scientific research. Aging is thought to be a passive process of cumulative damage and breakdown in organismal functioning. Longevity and aging may be influenced by, and in turn influence, mood disorders and response to stress, due to teleological evolutionary reasons or mundane lifestyle consequences. Compelling evidence suggests that mental state can affect health and longevity. It is presumed that this is mediated through behaviors that have favorable or detrimental health consequences.

Individuals with mood disorders and stress disorders have a significantly shorter life expectancy. Further, aging can lead to depression, attributable at least in part to physical health problems and related disability. Antidepressants have been shown to improve longevity in C. *elegans.* For example, the atypical anti-depressant mianserin, which is used for treating depression and stress disorders, has been shown by the inventors of the present disclosure to increase longevity in C. *elegans.*

The bi-directional relationship between mood, stress, and life expectancy may have a genetic basis, and be susceptible to therapeutic interventions. For example, targeting genes involved in the "life switch" that regulate the aging pathways and genes that can slow, pause, or reduce the effects of aging and/or increase life expectancy for therapeutic intervention have the potential to increase longevity and/or enhance quality of life in the later part of a subject's life. Targeting these genes further have the potential to treat subjects having diseases that affect life expectancy.

Accordingly, there exists a need for methods for identifying biological pathways involved in the active regulation by mood and stress of life expectancy. There further exists a need for methods for identifying therapeutics that affect biological pathways involved in mood, stress, and life expectancy. Identifying biological targets and drugs that affect these biological targets can further be used to increase longevity, prolong healthspan, and treat subjects having diseases that affect life expectancy such as diseases causing accelerated aging.

US 2011/207128 A1 relates to the association of gene expression patterns with familial longevity and survival. US 2009/083868 A1 relates to methods of identifying compounds that modulate longevity and/or delay onset of age-related diseases or conditions by modulating an activity or expression level of a wwp-1 or ubc-18 gene or polypeptide or other gene or protein involved in the dietary restriction pathway.

WO 2009/026116 A2 discloses a genome wide association study to construct a GeneMap for longevity. providesWO 2006/138696 A2 relates to methods for identifying agents that modulate the expression of a nucleic acid encoding different genes. US 2009/092551 A1 discloses a method of screening for a modulator of longevity.

### BRIEF DESCRIPTION OF THE DISCLOSURE

The present disclosure relates generally to analyzing pharmacodynamic effects of antidepressant treatments. More particularly, the present disclosure relates to methods for identifying biological pathways involved in active longevity, i.e., the active regulation of life expectancy by mood and stress. The present disclosure further relates to methods for identifying therapeutics that affect active longevity. The present disclosure also relates to uses of compounds for modulating active longevity genes.

In one aspect, the present disclosure is directed to a method of identifying a modulator of active longevity, the method comprising: providing a C. *elegans* animal; administering a candidate compound to the C. *elegans* animal; and monitoring expression of a C. *elegans* gene chosen from one or more genes in Tables 1, 2 and 3.

In another aspect, the present disclosure is directed to a compound chosen from one or more compounds in Tables 7, 8 and 9 for use in a method of modulating active longevity in a subject in need thereof, the compound administered to the subject; and monitoring expression of a gene chosen from one or more genes in Tables 1, 2 and 3, wherein a change in the expression of the gene indicates that active longevity is modulated.

In another aspect, the present disclosure is directed to a compound chosen from an omega-3 fatty acid, lithium, valproate, and combinations thereof for use in a method for modulating an active longevity gene in a subject in need thereof, the compound administered to the subject in need thereof; and monitoring expression of one or more genes chosen from Table 4, 5 and 6, wherein a change in the expression of the gene indicates that the active longevity gene is modulated.

In yet another aspect, the present disclosure is directed to a method for determining a biological age score in a subject, the method comprising: determining expression of one or more gene chosen from Tables 1, 2, 3 in a sample from the subject; computing a biological age score from the expression level by computing the Z-scores of the expression level of the one or more gene, wherein the calculation is gender specific; and identifying the subject as having propensity for active longevity if the biological age score is higher than average population levels for the chronological age of the subject.

In another aspect, the present disclosure is directed to a a method for determining propensity for dying in a subject, the method comprising: determining expression of one or more gene chosen from Tables 1, 2 and 3 in a sample from the subject; identifying the subject as having a propensity for dying by computing a probability of dying if the expression level or slope of change of the one or more gene are higher in the subject compared to the average levels or slope of change in individuals of the same chronological age in the general population.

### BRIEF DESCRIPTION OF THE DRAWINGS

FIG. 1 is a schematic depicting the possible "life switch" that actively regulates longevity versus dying.

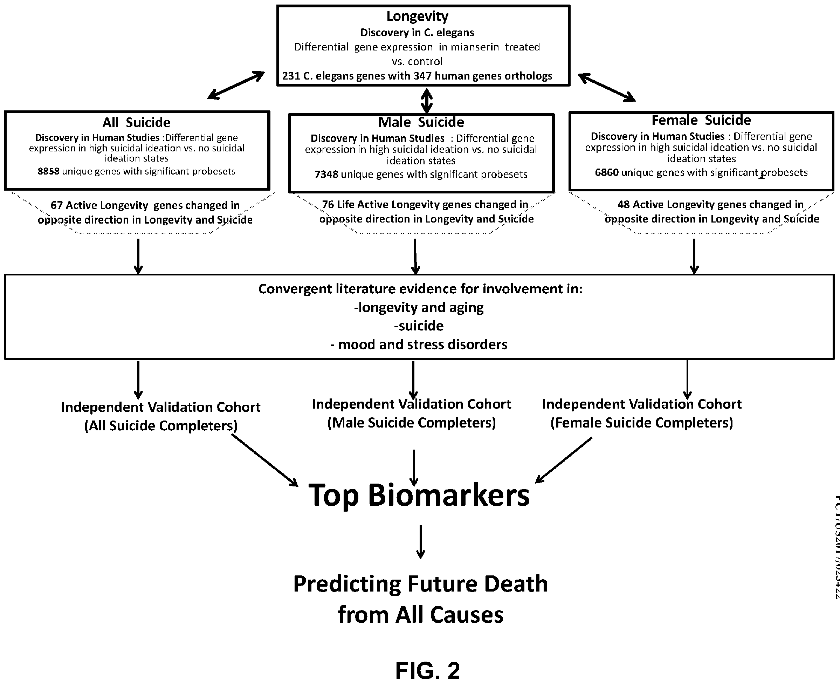

FIG. 2 is a flow chart depicting the experimental design for the discovery, prioritization, validation and testing to identify biomarkers for active longevity.
FIG. 3 are Venn diagrams depicting convergent evidence for active longevity biomarkers being at the intersection of longevity/aging, suicide, mood, and stress.

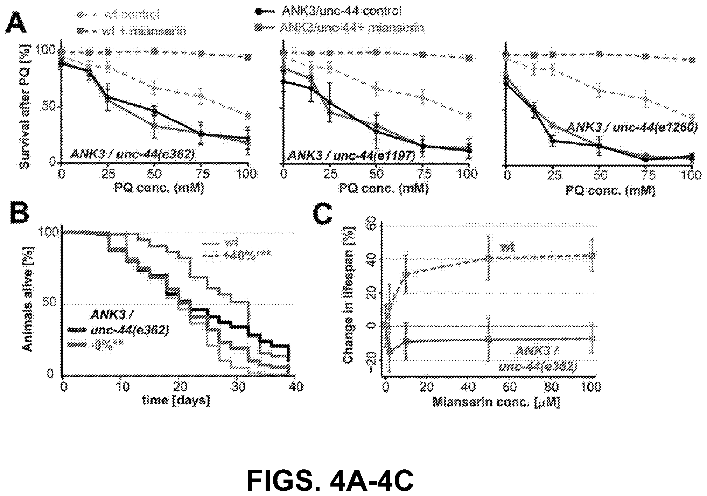

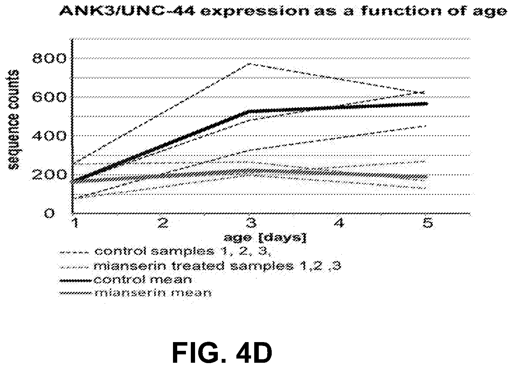

FIG. 4A are graphs depicting Mianserin-induced protection from oxidative stress requires ANK3/unc-44, the C. *elegans* homolog of mammalian ANK3. Wild-type (wt) N2 strain (dotted lines) or ANK3/unc-44 mutants (bold lines), at day 1 adult stage, were treated with water (black) or 50 uM Mianserin (red), followed by increasing concentrations of paraquat five days later. Survival of animals was determined 24 h after paraquat addition and plotted in [%] (Y-axis) as a function of paraquat concentration [mM] (X-axis). Parallel wt (N2) control experiments (dotted lines) are shown for each graph. Mianserin failed to increase resistance to oxidative stress in three independent alleles (e362, e1197, e1260) of ANK3/unc-44. All error bars show S.E.M for 3 to 4 independent experiments.
FIG. 4B is a graph depicting lifespan curves of wt and unc-44(e362) animals treated with water or 50 µM Mianserin. Graph shows animals alive [%] (Y-axis) as a function of time [days] (X-axis). Dotted lines represent wt (N2) animals and bold lines represent unc-44(e362) mutants. Black: solvent control; red: Mianserin 50 µM. In wild-type animals, Mianserin increases lifespan by +40%, while it does not (-9%) in unc-44(e362) mutant animals. Asterisks indicate P values (^{∗∗}; P<0.01, ^{∗∗∗}P<0.001).
FIG. 4C is a graph depicting mean increase in lifespan [%] (Y-axis) as a function of Mianserin concentration [IM] (X-axis). Solid red line represents unc-44(e362) animals. Dotted red line represents the parallel control experiment of Mianserin-treated wt (N2) animals. Error bars show standard deviation for experimental replicates. No lifespan extension is observed in ANK3 / unc-44(e362) mutants at any Mianserin concentration.
FIG. 4D is a graph depicting ANK3/unc-44 expression with age.
FIG. 5A is a graph depicting ANK3 expression in blood in psychiatric patients for predicting young age.
FIG. 5B is a graph depicting ANK3 expression in blood in people who committed suicide.
FIG. 5C is a table summarizing results by gender and diagnosis (Dx).
FIG. 6 is a flow diagram depicting a proposed mechanistic cascade.

### DETAILED DESCRIPTION

Unless defined otherwise, all technical and scientific terms used herein have the same meaning as commonly understood by one of ordinary skill in the art to which the disclosure belongs. Although any methods and materials similar to or equivalent to those described herein can be used in the practice or testing of the present disclosure, the preferred methods and materials are described below.

As used herein, "longevity" and "lifespan" (or "life span") refer to the length of a subject's life, for example, the number of years, months, weeks, days, minutes, etc., in the lifespan of an animal.

As used herein an "increase" or "modulation" of longevity includes a delay in the onset of age-related diseases and/or conditions and/or a delay and/or stabilization of the aging process.

A modulator is a compound that modulates expression and/or activity of a given gene, mRNA, protein, polypeptide, or the like, to produce a phenotypic change such as a change in lifespan or a delay in the onset of an age-related disease or condition. As used herein "modulate" refers to a change in an expression level, activity or property of the gene, protein, etc. For example, modulation can cause an increase or a decrease in a protein activity (e.g., catalytic activity) or binding characteristic (e.g., binding of a transcription factor to a nucleic acid). Modulation can cause an increase or decrease in expression of one or more genes, a change in transcription level, a change in stability of an mRNA that encodes a polypeptide, a change in translation efficiency, and/or a change in protein stability.

As used herein, "a reference expression level of a biomarker" refers to the expression level of a biomarker established for a subject with no mood disorder(s) and/or stress disorder(s), expression level of a biomarker in a normal/healthy subject with no suicidal ideation as determined by one skilled in the art using established methods as described herein, and/or a known expression level of a biomarker obtained from literature. The reference expression level of the biomarker can also refer to the expression level of the biomarker established for any combination of subjects such as a subject with no mood disorder(s) and/or stress disorder(s), expression level of the biomarker in a normal/healthy subject with no mood disorder(s) and/or stress disorder(s), and expression level of the biomarker for a subject who has no mood disorder(s) and/or stress disorder(s) at the time the sample is obtained from the subject, but who later exhibits mood disorder(s) and/or stress disorder(s). The reference expression level of the biomarker can also refer to the expression level of the biomarker obtained from the subject to which the method is applied. As such, the change within a subject from visit to visit can indicate modulation of a biomarker. A plurality of expression levels of a biomarker can be obtained from a plurality of samples obtained from the same subject and used to identify differences between the plurality of expression levels in each sample. Thus, in this embodiment, samples obtained from the subject can provide an expression level of a blood biomarker and a reference expression level of the blood biomarker.

As used herein, "expression level of a biomarker", "expression level of a gene" and "expression level of one or more gene" refer to the process by which a gene product is synthesized from a gene encoding the biomarker as known by those skilled in the art. The gene product can be, for example, RNA (ribonucleic acid) and protein. Expression level can be quantitatively measured by methods known by those skilled in the art such as, for example, northern blotting, amplification, polymerase chain reaction, microarray analysis, tag-based technologies (e.g., serial analysis of gene expression and next generation sequencing such as whole transcriptome shotgun sequencing or RNA-Seq), Western blotting, enzyme linked immunosorbent assay (ELISA), and combinations thereof.

Suitable subjects include non-human animals, such as, for example, nematodes, mammals, non-human primates, rodents (e.g., mice, rats, and hamsters), stock and domesticated animals (e.g., pigs, cows, sheep, horses, cats, and dogs), and birds. Suitable subjects also include humans.

As used herein, the terms "control", "control cohort", "reference sample", and "control sample" refer to a sample obtained from a source that is known, or believed, to not be afflicted with the disease or condition for which a method or composition of the present disclosure is being used to identify. The control can include one control or multiple controls. In one embodiment, a reference sample or control sample is obtained from an individual who is not the subject or patient in whom a disease or condition is being identified using a composition or method of the invention. In another embodiment, the reference sample or control sample is obtained from the same individual in whom a disease or condition is being identified using a composition or method of the present disclosure at a separate time period (e.g., 1 week earlier, 2 weeks earlier, 1 month earlier, 1 year earlier, and the like) as the test sample.

In one aspect, the present disclosure is directed to a method of identifying a modulator of longevity. The method includes: providing a C. *elegans* animal; administering a candidate compound to the *C. elegans* animal; and monitoring expression of a C. *elegans* gene selected from those provided in Table 1, wherein a change in the expression of the C. *elegans* gene indicates that the candidate compound modulates longevity. Expression level can be monitored by methods known by those skilled in the art such as, for example, northern blotting, amplification, polymerase chain reaction, microarray analysis, tag-based technologies (e.g., serial analysis of gene expression and next generation sequencing such as whole transcriptome shotgun sequencing or RNA-Seq), Western blotting, enzyme linked immunosorbent assay (ELISA), and combinations thereof.

Suitable biomarkers include those chosen from Tables 1-6. Other suitable biomarkers include those chosen from ankyrin 3 (ANK3), peptidylprolyl isomerase F (PPIF), superoxide dismutase 2 (SOD2), myosin, heavy chain 9 (MYH9), neural precursor cell expressed, developmentally down-regulated 4-like (NEDD4L), dihydrouridine synthase 4-like (DUS4L), cytochrome C oxidase subunit via polypeptide 1 (COX6A1), steroid-5-alpha-reductase, alpha polypeptide 1 (SRD5A1), cell division cycle 25B (CDC25B), and combinations thereof.

Suitable candidate compounds include antidepressants such as mianserin, mirtazapine, amoxapine, minaprine, and the like, and combinations thereof. Other suitable compounds include those chosen from Tables 7-9.

In another aspect, the present disclosure is directed to a compound chosen from one or more compounds in Tables 7, 8 and 9 for use in a method of modulating active longevity in a subject in need thereof, the compound administered to the subject; and monitoring expression of a gene chosen from one or more genes in Tables 1, 2 and 3, wherein a change in the expression of the gene indicates that active longevity is modulated. In an exemplary embodiment, a compound is chosen from an omega-3 fatty acid, lithium, mianserin ((±)-2-methyl-1,2,3,4,10,14b-hexahydrodibenzo [c,f]pyrazino [1,2-a]azepine), dicoumarol (3,3'-Methylenebis(4-hydroxy-2H-chromen-2-one)), diethylstilbestrol (4,4'-(3E)-hex-3-ene-3,4-diyldiphenol; (E)-11,12-Diethyl-4,13-stilbenediol), meglumine ((2R,3R,4R,5S)-6-(Methylamino)hexane-1,2,3,4,5-pentol), troglitazone ((RS)-5-(4-[(6-hydroxy-2,5,7,8-tetramethylchroman-2-yl)methoxy]benzyl)thiazolidine-2,4-dione), cyclopentolate((RS)-2-(dimethylamino)ethyl(1-hydroxycyclopentyl)(phenyl)acetate), mycophenolic acid ((4E)-6-(4-Hydroxy-6-methoxy-7-methy1-3-oxo-1,3-dihydro-2-benzofuran-5-yl)-4-methylhex-4-enoic acid), irinotecan ((S)-4,11-diethy1-3,4,12,14-tetrahydro-4-hydroxy-3,14-dioxo1H-pyrano[3',4' :6,7]-indolizino [1,2-b]quinolin-9-yl-[1,4'bipiperidine]-1'-carboxylate), metanephrine (4-(1-hydroxy-2-methylamino-ethyl)-2-methoxy-phenol), gliquidone (N-(cyclohexylcarbamoyl)-4-[2-(7-methoxy-4,4-dimethyl-1,3-dioxo-3,4-dihydroisoquinolin-2(1H)-yl)ethyl]benzenesulfonamide), nifedipine (3,5-dimethyl 2,6-dimethyl-4-(2-nitrophenyl)- 1,4-dihydropyridine-3,5-dicarboxylate), pioglitazone ((RS)-5-(4-[2-(5-ethylpyridin-2-yl)ethoxy]benzyl)thiazolidine-2,4-dione), terbutaline ((RS)-5-[2-(tert-butylamino)-1-hydroxyethyl]benzene-1,3-diol), capsaicin ((E)-N- [(4-Hydroxy-3-methoxyphenyl)methy1] -8-methylnon-6-enamide), homochlorcyclizine (1-[(4-chlorophenyl)-phenylmethyl]-4-methyl-1,4-diazepane), piracetam(2-(2-Oxopyrrolidin-1-yl)acetamide), minaprine (4-methyl-N-(2-morpholin-4-ylethyl)-6-phenylpyridazin-3-amine), quercetin (2-(3,4-dihydroxyphenyl)-3,5,7-trihydroxy-4H-chromen-4-one), rosiglitazone ((RS)-5-[4-(2-[methyl(pyridin-2-yl)amino]ethoxy)benzyl]thiazolidine-2,4-dione), ergocalciferol ((3β,5Z,7E,22E)-9,10-secoergosta-5,7,10(19),22-tetraen-3-ol), resveratrol ((E)-5-(4-hydroxystyryl)benzene-1,3-diol), sirolimus ((3S,6R,7E,9R,110R,12R,14S,15E,17E,19E,21S,23 S,26R,27R,34aS)-9,10,12,13,14,21,22,23,24,25,26,27,3 2,33,34,34a-hexadecahydro-9,27-dihydroxy-3-[(1R)-2-[(1S,3R,4R)-4-hydroxy-3-methoxycyclohexyl]-1-methylethyl]-10,21-dimethoxy-6,8,12,14,20,26-hexamethyl-23,27-epoxy-3H-pyrido[2,1-c][1,4]-oxaazacyclohentriacontine-1,5,11,28,29(4H,6H,31H)-pentone), estradiol ((8R,95,13S,14S,17S)-13-methyl-6,7,8,9,11,12,14,15,16,17-decahydrocyclopenta[a]phenanthrene-3,17-diol), amoxapine (2-chloro-11-(piperazin-1-yl)dibenzo [b,f] [1,4]oxazepine), quinacrine ((RS)-N'-(6-Chloro-2-methoxy-acridin-9-yl)-N,N-diethylpentane-1,4-diamine), sulfinpyrazone (1,2-dipheny1-4-[2-(phenylsulfinyl)ethyl]pyrazolidine-3,5-dione), and combinations thereof for use in the method for modulating a longevity gene in a subject in need thereof, wherein the compound is administered to the subject in need thereof.

The above mentioned use of the compound can further include monitoring expression of a gene selected from those provided in Tables 1-6, wherein a change in the expression of the gene indicates that the longevity gene is modulated.

The method can further include determining an expression level of the gene selected from those provided in Tables 1-6 in a sample.

Suitable samples for use in the methods of the present disclosure can include, for example, blood, a lymphoblastoid cell, cerebral spinal fluid, peripheral tissue, and the like, and combinations thereof.

The above mentioned use of the composition modulates expression of a biomarker in the subject. Suitable biomarkers that can be modulated are those provided in Tables 1-6.

In another aspect, the present disclosure is directed to a compound chosen from an omega-3 fatty acid, lithium, valproate, and combinations thereof for use in a method for modulating an active longevity gene in a subject in need thereof. The compound is administered to the subject in need thereof; and expression of one or more genes chosen from Table 4, 5 and 6 is monitored, wherein a change in the expression of the gene indicates that the active longevity gene is modulated.

In one embodiment, the compound is chosen from Table 7 and the gene is chosen from Table 1. In another embodiment, the subject is a male subject and wherein the compound is chosen from Table 8 and the gene is chosen from Table 2. In another embodiment, the subject is a female subject and wherein the compound is chosen from Table 9 and the gene is chosen from Table 3.

In yet another aspect, the present disclosure is directed to a method for determining a biological age score in a subject, the method comprising: determining expression of one or more gene chosen from Tables 1, 2, 3 in a sample from the subject; computing a biological age score from the expression level by computing the Z-scores of the expression level of the one or more gene, wherein the calculation is gender specific; and identifying the subject as having propensity for active longevity if the biological age score is higher than average population levels for the chronological age of the subject.

In one embodiment, the compound is chosen from Table 7 and the gene is chosen from Table 1. In another embodiment, the subject is a male subject and wherein the compound is chosen from Table 8 and the gene is chosen from Table 2. In another embodiment, the subject is a female subject and wherein the compound is chosen from Table 9 and the gene is chosen from Table 3.

In another aspect, the present disclosure is directed to a method for determining propensity for dying in a subject, the method comprising: determining expression of one or more gene chosen from Tables 1, 2 and 3 in the sample from the subject; identifying the subject as having a propensity for dying by computing a probability of dying if the expression level or slope of change of the one or more gene are higher in the subject compared to the average levels or slope of change in individuals of the same chronological age in the general population.

In one embodiment, the computing a probability of dying is by receiver operating curves area under the curve (ROC AUC), Cox Regressions, and combinations thereof. In one embodiment, the computing a probability of dying is by Cox Regressions. In one embodiment, the computing a probability of dying is by a combination of receiver operating curves area under the curve (ROC AUC) and Cox Regressions.

In one embodiment, the probability of dying is less than 7 years. In one embodiment, the probability of dying is from about 3 years to about 7 years.

Suitable samples include blood, a lymphoblastoid cell, cerebral spinal fluid, and a peripheral tissue.

In one embodiment, the one or more gene is chosen from Table 1. In another embodiment, the subject is a male subject and the one or more gene is chosen from Table 2. In another embodiment, the subject is a female subject and the one or more gene is chosen from Table 3. Particularly suitable subjects are human subjects.

In yet another aspect, the present disclosure is directed to use of a compound for modulating active longevity in a subject in need thereof comprising: administering a compound chosen from one or more compounds in Tables 7, 8 and 9 to the subject; and monitoring expression of a gene chosen from one or more genes in Tables 1, 2 and 3, wherein a change in the expression of the gene indicates that active longevity is modulated.

In one embodiment, the compound is chosen from Table 7 and the gene is chosen from Table 1. In another embodiment, the subject is a male subject and wherein the compound is chosen from Table 8 and the gene is chosen from Table 2. In another embodiment, the subject is a female subject and wherein the compound is chosen from Table 9 and the gene is chosen from Table 3. Particularly suitable subjects are human subjects.

Suitable samples include blood, a lymphoblastoid cell, cerebral spinal fluid, and a peripheral tissue.

In another aspect, the present disclosure is directed to use of a compound for modulating an active longevity gene in a subject in need thereof comprising: administering to the subject in need thereof a compound chosen from an omega-3 fatty acid, lithium, valproate, and combinations thereof; and monitoring expression of one or more genes chosen from Table 4, 5 and 6, wherein a change in the expression of the gene indicates that the active longevity gene is modulated.

In one embodiment, the one or more gene is chosen from Table 4. In another embodiment, the subject is a male subject and the one or more gene is chosen from Table 5. In another embodiment, the subject is a female subject and wherein the one or more gene is chosen from Table 6. Particularly suitable subjects are human subjects.

### EXAMPLES

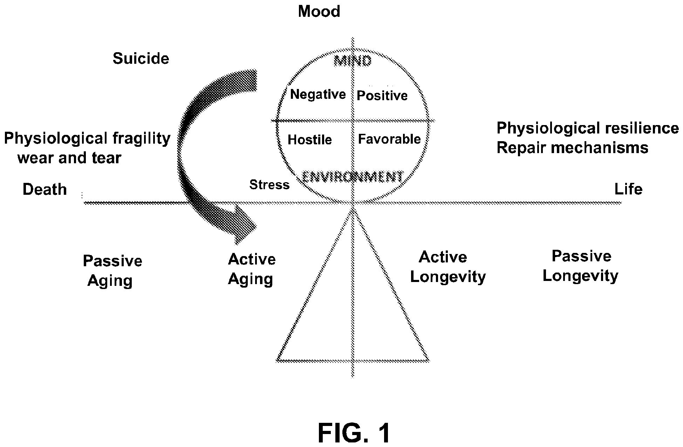

Aging and dying are thought to be passive processes of cumulative damage and breakdown in organismal functioning, with the exception of suicide, which is an active form of dying. Compelling evidence suggests that mental state can affect health and longevity. It is presumed that the effect of mental state on health and longevity is mediated through behaviors that have favorable or detrimental health consequences. Previously conducted translational studies from C. *elegans* to humans were conducted to identify genes and blood biomarkers involved in mood and stress-modulated longevity. Separate human studies were conducted to identify genes and blood biomarkers involved in suicide. An intriguing overlap between these two studies was identified. First, some of the same biomarkers were involved in longevity and in suicide, but with gene expression changed in opposite directions. Second, biological pathways related to cellular viability were involved in both cases. Third, drug repurposing analyses identified as suicidality treating compounds agents that are being currently studied for longevity. These results indicated the possible existence of a "life switch" that actively regulates longevity vs. dying (FIG. 1).

As described in the Examples, genes were identified that were changed in expression in an opposite direction in a C. *elegans* longevity model and a comprehensive study of suicidality, using the overall data as well as the data separated by gender. These genes were prioritized using Bayesian-like Convergent Functional Genomics (CFG) platform, using other published evidence in the field, for involvement in: (1) longevity and aging, (2) suicide, and (3) mood disorders and stress. The prioritized active longevity biomarkers were validated for change in opposite direction in suicide completers. The diagnostic/prognostic ability of the biomarkers was examined in an independent cohort of psychiatric patients, who were subject to intense negative mood and stress. The ability of the levels of the biomarkers, and their slope of change between visits, was determined to predict future death by any cause. Mental health and non-mental health drugs were identified that act on individual biomarkers involved in active longevity. Compounds were bioinformatically repurposed using the gene expression signature of biomarkers for active longevity. All the above analyses were also conducted separately in males and females to determine the best repurposed drugs, pharmacogenomics results, and predictive biomarkers for each gender.

### Convergent Functional Genomics

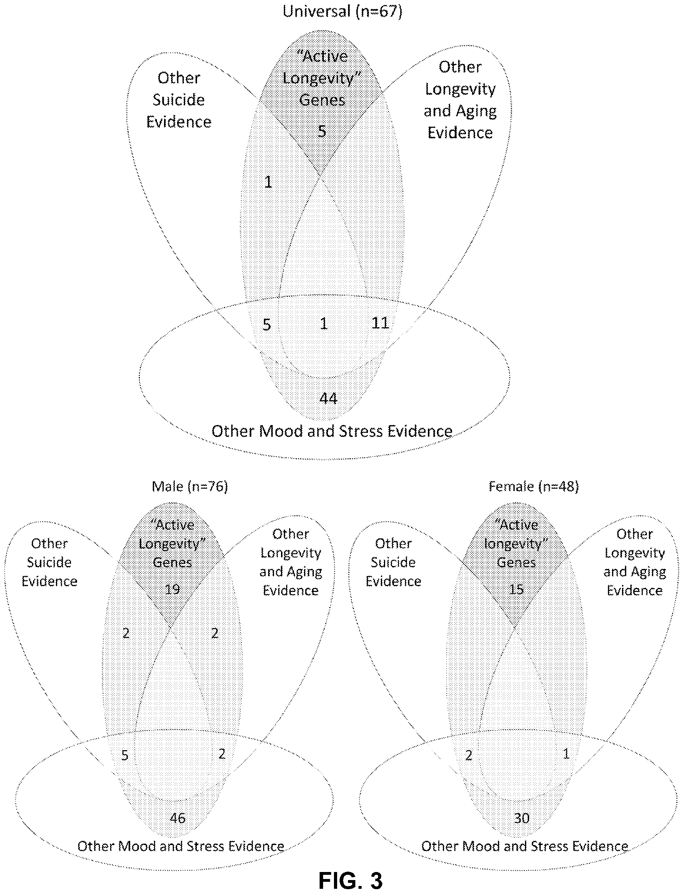

*Databases.* Manually curated databases of all the human gene expression (postmortem brain, blood and cell cultures), human genetics (association, copy number variations and linkage), and animal model gene expression and genetic studies published to date on psychiatric disorders were established (Laboratory of Neurophenomics, Indiana University School of Medicine, www.neurophenomics.info). Only the findings deemed significant in the primary publication, by the study authors, using their particular experimental design and thresholds, are included in the databases. The databases include only primary literature data and do not include review papers or other secondary data integration analyses to avoid redundancy and circularity. These large and constantly updated databases were used in the CFG cross and prioritization (FIGS. 3A & 3B). For this Example, data from 1556 papers on mood and on stress were present in the databases at the time of the CFG analyses (February 2015) (human: genetic studies-761, brain studies-226, peripheral fluids- 311; non-human• genetic studies-41, brain studies-195, peripheral fluids-22).

*Human postmortem brain gene expression evidence.* Converging evidence was scored for a gene if there were published reports of human postmortem data showing changes in expression of that gene or changes in protein levels in brains from participants with mood or stress disorders.

*Human blood and other peripheral tissue gene expression data.* Converging evidence was scored for a gene if there were published reports of human blood, lymphoblastoid cell lines, CSF, or other peripheral tissue data showing changes in expression of that gene or changes in protein levels in participants with mood or stress disorders.

*Human genetic evidence (association and linkage).* To designate convergence for a particular gene, the gene had to have independent published evidence of association or linkage for mood disorders or stress disorders. For linkage, the location of each gene was obtained through GeneCards (www.genecards.org), and the sex-averaged cM location of the start of the gene was then obtained through compgen.rutgers.edu/mapinterpolator. For linkage convergence, the start of the gene had to map within 5 cM of the location of a marker linked to the disorder.

*Animal model brain and blood gene expression evidence.* For animal model brain and blood gene expression evidence, prior datasets 6-8 (Ogden et al. Molecular psychiatry 2004; 9(11): 1007-1029; Le-Niculescu et al. Am. J. Medical Genetics Part B, Neuropsychiatric genetics: the official publication of the International Society of Psychiatric Genetics 2008; 147B(2): 134-166; Le-Niculescu et al. Translational Psychiatry 2011; 1: e4), as well as published reports from the literature curated in the databases.

*Animal model genetic evidence.* To search for mouse genetic evidence (transgenic and QTL) for candidate genes, PubMed as well as the Mouse Genome Informatics (www.informatics.jax.org; Jackson Laboratory, Bar Harbor, ME, USA) database, were searched using the keywords "mood, bipolar, depression, stress". For QTL convergence, the start of the gene had to map within 5 cM of the location of these markers.

*CFG scoring.* For CFG analysis, the external cross-validating lines of evidence were weighted such that findings in human postmortem brain tissue, the target organ, were prioritized over peripheral tissue findings and genetic findings, by giving them twice as many points. Human brain expression evidence was given 4 points, whereas human peripheral evidence was given 2 points, and human genetic evidence was given a maximum of 2 points for association, and 1 point for linkage. The scoring for the corresponding non-human lines of evidence were half of those in human (genetic-1 point, brain-2 points, peripheral-1 point). Each line of evidence was capped such that any positive findings within that line of evidence resulted in maximum points, regardless if it came from mood or stress (as the two may be interrelated in some studies), and regardless of how many different studies supported that single line of evidence, to avoid potential popularity biases. In addition to the external CFG score, genes were also prioritized based upon the internal score from the discovery analyses used to identify them, in mianserin treated C. *elegans* and in the suicide studies (Niculescu et al. 2017 in press). Genes identified in the discovery could receive a maximum of 8 points (4 from C. *elegans,* 4 from suicide).

The scoring system was decided upon before the analyses were carried out. More weight was given to the external score than to the internal score in order to increase generalizability and avoid fit to cohort of the prioritized genes. It is believed that this scoring system provides a good separation of genes based on internal discovery evidence and on external independent cross-validating evidence in the field. With multiple large datasets, machine learning approaches could be used and validated to assign weights to CFG.

### Gene expression studies

All Affymetrix microarray data was imported as .cel files into Partek Genomic Suites 6.6 software package (Partek Incorporated, St Louis, MI, USA). Using only the perfect match values, a robust multi-array analysis (RMA) was run, background corrected with quantile normalization and a median polish probe set summarization, to obtain the normalized expression levels of all probe sets for each chip. RMA was performed independently for each of the diagnoses used in the study to avoid potential artifacts due to different ranges of gene expression in different diagnoses. Then the participants' normalized data were extracted from these RMAs and assembled for the different cohort analyses. Gene expression data was then z-scored by gender and diagnosis, to avoid potential artifacts due to different ranges of gene expression in different gender and diagnoses when combining cohorts, and to be able to combine different markers into a panel.

### Statistical analyses

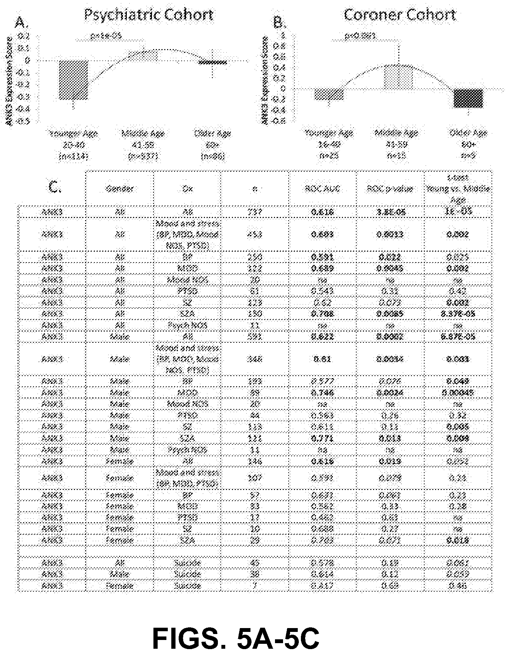

Receiver-operating characteristic (ROC) analyses were calculated using the pROC function of the R studio, and double-checked using IBM SPSS Statistics 21. Diagnosis was converted to a binary call of 0 (Middle and Old Age, above 40 years old) or 1 (Young Age, 20-40 years old) and entered as the state variable, with gene expression levels entered as the test variable. Additionally, Student's t-test was performed between young age (20-40 years old) and middle age (40-60 years old). For the top 9 genes, a Pearson R correlation (one-tail) was calculated between age and biomarker levels. Probesets that correlated inversely with age were selected, in 5 genes. On the Affymetrix HG-U133 Plus 2.0 GeneChip, there were 5 probesets in ANK3, and 25 probesets total in the top 9 genes. 4 probesets in ANK3 correlated with age compared with their direction of change in the mianserin-treated worms data, i.e., if a gene was increased in expression in mianserin-treated worms, it should correlate inversely with age; if a gene was decreased in expression in mianserin-treated worms, it should correlated directly with age. The single best correlated probeset in ANK3 was selected for future analyses. (FIGS. 5A-5C)

### Pathway analyses

IPA (Ingenuity Systems, www.ingenuity.com, Redwood City, CA, USA), GeneGO MetaCore (Encinitas, CA, USA), and Kyoto Encyclopedia of Genes and Genomes (KEGG) (through the Partek Genomics Suite 6.6 software package) were used to analyze the biological roles, including top canonical pathways, and diseases of the candidate genes resulting from the analysis, as well as to identify genes in the data set that were the target of existing drugs. The pathway analyses was run together for all the 347 human orthologs, then for those having some evidence for the human GWAS data (n=134).

### Connectivity Map analyses

To elucidate which drugs may induce a gene expression signature similar to the active longevity biomarkers from these results, the Connectivity Map v2 (also known as cmap) was used, which comprises a collection of genome-wide transcriptional expression data from cultured human cells treated with bioactive small molecules and simple pattern-matching algorithms that together enable the discovery of functional connections between drugs, genes and diseases through the transitory feature of common gene-expression changes. The cmap (www.broad.mit.edu/cmap) contains more than 7,000 expression profiles representing 1,309 compounds. The Affymetrix website was used to obtain the probesets ID corresponding to the Active Longevity candidate genes in the HGU133A array chip that cmap used. The quick query selection was used and the increase in expression genes probe set id was uploaded in the up tag file, and the decreased in expression genes probe set id was uploaded in the down tag file.

### EXAMPLE 1

In this Example, human orthologs of genes that were changed in expression by mianserin treatment in C. *elegans* were analyzed and biological pathways involved in longevity were identified.

Mianserin treated C. *elegans* whole-genome transcriptomic data was obtained as described (Rangaraju et al., eLife 2015; 4). 6701 genes were changed in expression with at least nominal significance (p< 0.05, FDR<10%) in mianserin vs. water treated worms. To ensure stringency in the analyses, a Bonferroni correction was applied to p-values for number of genes in the genome (21,035 genes) (Hillier et al. 2015), and carried forward in the analyses genes with a p-value of less than 2.4 x 10⁻⁶. 1068 worm genes survived correction. Out of these, 971 were consistently upregulated or downregulated at the three time-points tested (days 3, 5 and 10). (FIGS. 4A-4D).

To identify human orthologs corresponding to the 971 C. *elegans* genes, OrthoList (www.greenwaldlab.org/ortholist/), which is a compendium of four orthology prediction programs, and a manual search were conducted. To ensure reliability, genes having at least two out of the four prediction programs agreeing on assignments to human orthologs were retained. Out of 971 worm genes, 231 satisfied the above criteria. There were 347 human orthologs corresponding to these 231 worm genes. GeneCards (www.genecards.org) was used to confirm the gene symbol, name and chromosomal location.

An internal score was assigned to the 971 Bonferroni corrected *C.elegans* genes according to the distribution of p-values. The top 0.1% genes received an internal score of 4, top 5% received a score of 2 and the remaining C. *elegans* genes received a score of 1. The corresponding human orthologs received the same scores assigned to their C. *elegans* counterparts.

Out of 971 C. *elegans* genes that survived Bonferroni correction as being consistently differentially expressed, there were 347 human orthologs for the 243 C. *elegans* genes that were assigned with a high degree of certainty (concordance of at least two different orthology identifying software packages/databases). Studies on potential suicide biomarkers identified 8867 genes differentially expressed with a low stringency threshold. In a discovery sub-analysis by gender, there were 7348 genes in males, and 6860 genes in females.

For the primary universal (males and females) analysis, the overlap between the C. elegans-derived 347 genes and human suicide 8867 genes yielded 67 genes changed in expression opposite direction (19.3% of the C. *elegans* longevity genes), and 43 genes changed in expression in the same direction. That is an approximately 1.6-fold enrichment/odds-ratio for opposite direction of change. For the secondary analyses, the overlap between the C. *elegans*-derived genes and human male suicide 7348 genes yielded 76 genes changed in expression in opposite direction (21.9% of the C. *elegans* longevity genes), and 46 genes changed in expression in the same direction. That is an approximately 1.7-fold enrichment/odds-ratio for opposite direction of change. The overlap between the C. elegans-derived genes and human female suicide 6860 genes yielded 48 genes changed in expression in opposite direction (13.8% of the C. *elegans* longevity genes), and 58 genes changed in expression in the same direction. There was no enrichment/odds-ratio for opposite direction of change.

Convergent functional genomics ("CFG") prioritization was conducted using prior published human data in the field, for the 67 genes in the primary overall universal analysis, and the lists of 76 genes in the secondary male analysis and 48 genes in the secondary female analysis. Genes received a maximum of 8 points from internal evidence (4 from C. *elegans,* 4 from suicide studies), as well as 8 points for external literature evidence for longevity and aging, 8 points for external literature evidence for suicide, and 8 point for external literature evidence for mood and stress.

Genes with a CFG Score of 6 and above were tested to identify if they were stepwise changed in expression in the blood of a cohort of individuals with suicidal ideation (SI) and a cohort of suicide completers. Of the genes that were stepwise changed from no SI to high SI to suicide completion, some were nominally significant in ANOVA, and a number of biomarkers that were Bonferroni significant (i.e., survived correction for number of biomarkers assessed): 18 genes in the universal analysis, 18 genes in the male analysis, and 6 genes in the female analysis.

A subset of biomarkers that had the strongest evidence from discovery, from prioritization, or from validation were identified: 9 genes in the universal analysis, 10 genes in the male analysis, and 8 genes in the female analysis (Tables 1-3).

**Table 1. Universal Biomarkers for Active Longevity.**

| **Human Gene Symbol/ Gene Name** | **Discove ry C. elegans Longevi ty (Direct Change) Transcr ipt ID P-value/Sc ore** | **Discove ry Human Suicide (Directi on of Change) Affymet rix Probese tID Method/ Score** | **Prioritiz ation CFG Score for Longevi ty and Aging** | **ion of Prioritiz ation CFG Score for Suicide** | **Prioritiz ation CFG score for Mood and Stress** | **Total Discove ry and Prioritiz ation CFG score** | **Validati on in suicide complet ers ANOVA p-value** | **Top Biomar ker from** | **Top Predicto r for Future Death from All Causes** |
|---|---|---|---|---|---|---|---|---|---|
| **ACADS** Bacyl-CoAdeh ydrogen ase, short/bra nched chain | (I) (acdh-1) C55B7.4 1.50E-13 /4 | (D) 205355_ at DE/1 | 0 | 0 | 5 | 10 | 4.52E-07 | Discover y | |
| **TRPA1** transient receptor potential cation channel, subfamil y A, member 1 | (D) (trpa-1) C29E6.2 1.10E-07 /2 | (I) 208349_ at AP/2 | 0 | 0 | 0 | 4 | NS | Discover y | |
| **GFPT1** glutamin e-fructose-6-phosphat e trans ami nase1 | (I) F22B3.4 6.00E-14/4 | (D) 227027_ at/2 | 0 | 0 | 6 | 12 | NS | Discover y and Prioritiz ation | |
| **CD109** CD 109 molecule | (D) (tep-1) ZK337.1 3.10E-11/3 | (I) 226545_ at DE/2 | 0 | 2 | 2 | 9 | 2.16E-09 | Discover y and Validati on | |
| **ANK2** ankyrin 2, neuronal | (D) (unc-44) B0350.2 1.00E-06/1 | (I) 202920_ at DE/1 | 6 | 0 | 6 | 14 | 4.37E-09 | Prioritiz ation | |
| **DBH** dopamin e betahydr oxylase (dopami ne betamonoox ygenase) | (I) (tbh-1) H13NO 6.6 8.50E-09/3 | (D) 234916_ at DE/2 | 0 | 6 | 2 | 13 | NS | Prioritiz ation | |
| **KIF3C** kinesin family member 3C | (D) (klp-11) F2005.2 2.30E-06/1 | (I) 203389_ at AP/2 | 0 | 0 | 5 | 8 | NS | Prioritiz ation | 5 Year Death Based on Levels ROC AUC 0.64/p-value 0.0016 |
| **PRSS33** protease serine, 33 | (I) (try-1) ZK546.1 5 1.20E-08/2 | (D) 1552348 at AP/2 | 0 | 0 | 3 | 7 | 311E-11 | Validati on | 5 Year Death Based on Slope ROC AUC 0.61/p-value 0.037 |
| | | | | | | | | | 5 Year Death Based on Levels and Slope ROC AUC 0.60/p-value 0.047 |
| | | | | | | | | | All Future Death Based on Slope Cox Regressi on HR 1.46/p-value 0.043 |
| | | | | | | | | | All Future Death Based on Levels and Slope Cox Regressi on HR 1.63/p-value 0.025 |
| **YIPF5** Yipl domain family, member 5 | (I) F32D8.1 4 1.40E-06 /1 | (D) 224949_ at DE/2 | 0 | 1 | 5 | 9 | 2.15E-14 | Validati on | |
| **PARL** presenili nassociat ed, rhomboi d-like | (I) (rom-5) Y54E1 0A. 14 2.00E-06/1 | (D) 218271_ s_at DE/1 | 2 | 0 | 4 | 8 | 3.66E-10 | | 7 Year Death Based on Levels and Slope ROC AUC 0.61/p-value 0.028 |
| **PEBP1** phosphat idyletha nolamin e binding protein 1 | (I) 40A.3 1.70E-07/1 | (D) 205353_ s_at DE/1 | 0 | 0 | 5 | 7 | NS | | 3 Year Death Based on Levels ROC AUC 0.67/p-value 0.0016 |
| | | | | | | | | | 7 Year Death Based on Levels AUC 0.67/p-value 0.00012 |
| | | | | | | | | | All Future Death Based on Levels Cox Regressi on HR 1.47/p-value 0.0053 |
| **CYB5R** 2 cytochro meb 5 reductas e 2 | (I) (hpo-19) TO5H4. 51.20E-06/1 | (D) 220230_ s_at DE/1 | 0 | 4 | 0 ROC | 6 | NS | | 3 Year Death Based on Slope AUC 0.63/p-value 0.033 |
| | | | | | | | | | 3 Year Death Based on Levels and Slope ROC AUC 0.66/p-value 0.012 |

D- Decreased, I- Increased. AP- Absent/Present DE- Differentia Expression; Validation: Bold-Bonferroni significant; italic-nominally significant; NS- non-stepwise changed in validation. Prediction of death if the direction of change in expression was the same as in suicide, i.e. opposite of that in longevity.

**Table 2. Biomarkers for Active Longevity in Males.**

| **Human Gene Symbol /Gene Name** | **Discove ry** C.elega ns Longevi ty (Directi on of Change ) Transcri pt ID P-value/S core | **Discove ry** Human Suicide (Directi on of Change ) Affyme trix Probese t ID Method /Score | **Prioriti zation** CFG Scor e for Long evity and Aging | **Prioriti nation** CFG Score for Suicide | **Prioriti zation** CFG score for Mood and Stress | **Total Discove ry and Prioriti zation CFG score** | **Validat ionin suicide comple ters** ANOV A p-value | **Top Biomar ker from** | **Top Predict or for Future Death from All Causes** |
|---|---|---|---|---|---|---|---|---|---|
| **SCP EP1** Serine Carboxe pyptidas e 1 | (I) Y32F6 A.5 1.80E-11/3 | (D) 156066 Sat AP/4 | 0 | 0 | 2 | 9 | AP 4.10E-02 | Discove ry | |
| **GAP DH** Glycera ldehyde -3-Phospha te Dehydr oge nase | (I) (gpd-1) F33H1. 2 1.60E-08/4 | (D) 217398 _x_at DE/2 213453 _x_at DE /1 AFFXH UMGA PDH/M 33197 _5_at DE /1 AFFX-HUMG APDH/ M3319 7_M_at DE/1 | 0 | 0 | 6 | 12 | NS | Discove ry | |
| **NAV 3** Neuron Navigat or 3 | (D) (unc-53) F45E10 .1 1.30E-10/3 | (I) 204823 at DE /2 155265 8_a at DE /1 | 0 | 0 | 5 | 10 | NS | Discove ry | 3 Year Death Based on Levels ROC AUC 0.67/p-value 0.0027 |
| **FAM** 184A Family With Sequenc e Similari ty 184 Mem ber A | (D) (ta-278) CO2F1 2.7 2.00E-12/3 | (I) 155852 3_at AP/1 | 0 | 0 | 4 | 8 | NS | Discove ry and Prioritiz ation | |
| **ANK 3** Ankyrin 3 | (D) (unc-44) B0350. 2 1.00E-0 6/1 | (I) 207950 _s_at DE/2 | 2 | 0 | 8 | 13 | NS | Prioritiz ation | |
| **CTSB** Catheps in B | (I) F57F5. 1 2.10E 28/4 | (D) 200838 at DE /1 | 4 | 0 | 7 | 16 | 1.16E-0 1 | Prioritiz ation | |
| **GFPT** 1 Glutami ne-Fruct ose-6-Phospha te Trans aminase 1 | (I) F22B3. 4 6.00E-14/4 | D) 227027 _at AP/1 | 0 | 0 | 6 | 11 | 7.67E-0 1 | Prioritiz ation | |
| **CD10** 9 CD10 9 Mole cule | (D) (tep-1) ZK337. 1 3.10E-11/3 | (I) 226545 _at DE /1 | 0 | 2 | 2 | 8 | 4.98E-0 6 | Validati on | |
| **PRSS** 33 Protease Sevine 33 | (I) (try-1) ZK546. 15 1.20E-0 8/2 | (D) 15 8 at 5234 AP/2 DE/1 | 0 | 0 | 3 | 7 | DE 7.86E-0 7 AP 5.80E-0 8 Validati on | Validati on | |
| **YIPF** 5 Yipl Domain Family Member 5 | (I) F32D8. 14 1.40E-06/1 | (D) 224949 _at DE /1 221423 _s_at DE /1 | 0 | 1 | 5 | 8 | 1.09E-1 1 2.33E-07 | Validati on | |
| **CYB 5R2** Cytoc hrome B5 Reducta se 2 | (I) (hpo-19) TO5H 4.5 1.20E-06/1 | (D) 220230 s at DE/1 | 0 | 4 | 0 | 6 | NS | | 3 Year Death Based on Slope ROC AUC 0.64/p-v alue 0.038 3 Year Death Based on Slope and Levels ROC AUC 0.65/p-value 0.028 |
| **MSH 2** MutS Homo log 2 | (I) (msh-2) H26D2 1.2 4.40E-08/2 | (D) 209421 _at DE/1 | 0 | 0 | 5 | 8 | 1.00E-0 6 | | All Future Death Based on Levels Cox Regress ion HR 1.57/p-v alue 0.0049 |
| **MYH 8** Myosin Heavy Chain 8 | (D) (myo-3) K12F2. 1 1.60E-09/3 | (I) 34471_ a DE/1 | 0 | 0 | 1 | 5 | NS | | 7 Year Death Based on Levels ROC AUC 0.64/p-v alue 0.0022 |
| **NLGN 2** Neuroli gin 2 | (D) (nlg-1) C40C9 5.1.10E 06/1 | (I) 155442 8 s at AP/1 235838 _at DE/1 | 0 | 0 | 2 | 4 | NS | | 5 Year Death Based on Levels and Slope ROC AUC0 63/p-val ue 0.027 |
| **PARL** Presenil in Associa ted Rhom boid Like | (I) from-5) Y54E1 0A. 14 2.00E-06/1 | (D) 218271 s at DE/1 | 0 | 0 | 4 | 6 | 9.87E-08 | | 7 Year Death Based on Levels and Slope ROC AUC 0.64/p-value 0.012 |
| **PPIA (I)** Peptidyl prolyl Isome rase A | (I) (cyn-7) Y75B1 2B.2 7.20E-10/3 | (D) 211378 _x_at DE/1 | 0 | 0 | 7 | 11 | NS | | 5 Year Death Based on Levels ROC AUC 0.65/p-v alue 0.0018 |

D- Decreased, I- Increased. AP- Absent/Present, DE- Differential Expression; Validation: Bold-Bonferroni significant; italic-nominally significant; NS- non-stepwise changed in validation. Prediction of death if the direction of change in expression was same as in suicide, i.e. opposite of that in longevity.

**Table 3. Biomarkers for Active Longevity in Females.**

| **Human Gene Symbol/ Gene Name** | **Discove ry C.elega ns Longevi ty (Directi on of Change) Transcr ipt ID P-value/Sc ore** | **Disc overy Human Suicide (Dire ction of Change) Affymet rix Probese t ID Method/ Score** | **Prior itization CFG Score for Longevi ty and Aging** | **Prioritiz ation CFG Score for Suicide** | **Prioritiz ation CFG score for Mood and Stress** | **Total Discove ry and Prioriti zation CFG score** | **Validati on in suicide complet ers ANOVA p-value** | **TopBio marker from** | **Top Predicto r for Future Death from All Causes** |
|---|---|---|---|---|---|---|---|---|---|
| **ADAM1 2** ADAM ptidase Domain 12 | Metallop eAP/1 (D) (adm-2) CO4A1 1.4 2.90E-08/2 | (I) 2137 90_at 2137 90 at DE/1 | 0.00 | 0.00 | 2.00 | 5 | NS | Discov ery | All Future Death Based on Levels Cox Regressi on HR 2.26/p-value 0.039 |
| **NAV3** Neuron N avigato r 3 | (D) (unc-53) F45E10. 1 1.30E-10/3 | (I) 1562234 a_at DE/2 | 0.00 | 0.00 | 5.00 | 10 | NS | Discov ery | |
| **GFPT1** Glutami neFructose 6-Phosphat e Transam inase 1 | (I) F22B3. 4 6.00E-14/4 | (D) 227027 at AP/2 227027 at DE/2 | 0.00 | 0.00 | 6.00 | 12 | NS | Discover y and Prioriti zation | |
| **RAB14** RAB14, Member RAS Oncogen e Family | (D) (rab-14) K09A9. 2 8.20E-08/2 | (I) 2115 03_s at DE/2 | 1.00 | 0.00 | 6.00 | 11 | NS | Prioriti zation | |
| **JPH1** Junctoph ilin 1 | (D) T22C1.7 7.30E-08/2 | (I) 533_155 3 at AP/1 | 0.00 | 0.00 | 5.00 | 8 | 5.043E | Prioriti andzatio n Validati on | |
| **CD109** CD 109 Molecul e | (D) (tep-1) ZK337.3 .10E-11/ 3 | (I) 2265 45_at DE/1 | 0.00 | 2.00 | 2.00 | 8 | 4.08E-0 5 | Valida tion | |
| POLH Polymer ase Eta | (I) (polh-1) F53A3.2 7.30E-08/2 | TE0 2338 5 2_at AP/1 | 0.00 | 0.00 | 4.00 | 7 | 1.39E-02 | Validati on | |
| **SLC35B** 3 Solute Carrier Family 35 Member B3 | (I) (pst-2) F54E7.1 2.10E-08/2 | (D) 231003_ at DE/2 | 0.00 | 0.00 | 5.00 | 9 | 9.21E-0 5 | Valida tion | |
| **FBN2** Fibrillin 2 | (D) (mua-3) KO8E5. 3 7.70E-07/1 | (I) 203184_ at DE/1 | 0.00 | 0.00 | 4.00 | 6 | NS | | 5 Year Death Based on Levels ROC AUC 0.80/p-value 0.011 5 Year Death Based on Levels and Slope ROC AUC 0.80/p- |
| | | | | | | | | | value 0.027 |
| **MYH10** Myosin Heavy Chain 10 | (D) (nmy-1) F52B10 8.90E-07/1 | (I) 213067_ at DE/1 | 0.00 | 0.00 | 6.00 | 8 | *4.41E-*03 | | 7 Year Death Based on Levels ROC AUC 0.86/p-value 0.0046 |
| **PGRMC1** Progeste rone Receptor Membra ne Compon ent 1 | (I) (vem-1) K07E3.8 6.60E-08/2 | (D) 2011 20_s_at DE/1 201121 s at DE/1 | 0.00 | 0.00 | 6.00 | 9 | 6.79E-04 | | 7 Year Death Based on Levels and Slope ROC AUC 0.81/p-value 0.039 |
| SCOC Short Coiled-Coil Protein | (I) (unc-69) TO7A5. 6 2.40E-06/1 | (D) 2233 41_s_at DE/2 2247 86_at AP/2 2233 41_s_at AP/1 | 0.00 | 0.00 | 7.00 | 10 | NS | | 3 Year Death Based on Levels ROC AUC 0.85/p-value 0.022 |

D-Decreased, I- Increased. AP- Absent/Present DE-Differential Expression; Validation: Bold-Bonferroni significant; italic-nominally significant; NS- non-stepwise changed in validation. Prediction of death if the direction of change in expression was the same as in suicide, i.e. opposite of that in longevity.

### EXAMPLE 2

In this Example, levels of expression of individual biomarkers or groups of biomarkers, comparing those levels with the average levels in the reference population (everybody, men, women), or comparing to previous levels of the biomarker(s) in the person, including to examine the trend (slope of change) were determined for risk of future death. A change in expression in the opposite direction of longevity would be predictive of increased risk of future death.

Active longevity biomarker levels of expression and slope of change in expression between visits were correlated with the outcome "future death from all causes" in a cohort of psychiatric patients that had been followed longitudinally in the lab and through electronic medical records, to identify if the levels of expression at the time of their visits predicted future death. Cox regression Hazard Ratio (HR) and p-value were examined for all future follow-up in those who died vs. those who did not die. Biomarker levels or slopes or combinations of the two were also examined to identify whether these could predict who died from those that had at least 3, 5 and 7 years of follow-up, using Receiver Operating Curves Areas under the Curve (ROC AUC) and its p-value. Out of the complete lists of biomarkers (universal 67 genes, males 76 genes, females 48 genes), biomarkers were identified that were at least nominally significant p-value in the Cox Regression or ROC, and from those that had the best HR or AUC were identified (Tables 1-3). These results demonstrate that a person could be diagnosed for risk of dying using a combination of biomarkers, for example one or more predictive biomarkers from the universal list, and one or more predictive biomarkers from the gender list (male or female).

### EXAMPLE 3

In this Example, medications and natural compounds known for treating mental disorders and to prevent suicide were examined by database searches to identify if they have evidence modulating the expression of the biomarkers in the direction of longevity.

A bad state of mind, reflecting either a bad life and/or mental health issues, can lead to switching off of the "life switch", from the direction of active longevity to the direction of suicide. Omega-3 fatty acids, lithium, clozapine, other psychiatric medication were examined

As summarized in Tables 4-6, such individual biomarker-medication pairings can be used to identify which individuals should receive what drug (companion diagnostics, targeted therapeutics), and to monitor if they respond to treatment (pharmacogenomics).

**Table 4. Universal Biomarkers for Active Longevity - Pharmacogenomics for potential stratification and monitoring response to treatment. Biomarker genes that are targets of existing drugs and modulated by them in the same direction as longevity.**

| **Human Gene Symbol/G ene Name** | **Longevity Direction of Change** | **Suicidalit y Direction of Change** | **Modulate d by Omeg a-3** | **Modulate d by Lithium** | **Modulate d by Clozapine** | **Modulate d by Other Psychiatr ic Drugs** | **Modulate d by Other Non-Psyc hiatric Drugs** |
|---|---|---|---|---|---|---|---|
| **ANK3** ankyrin 3, node of Ranvier (ankyrin G) | D | I | Yes | | | | |
| **ASPSCR 1** alveolar soft part sarcoma chromoso me region, candidate 1 | I | D | Yes | | | | |
| **CBS** cystathion ine-beta-synthase | I | D | Yes | | | | |
| **COX6A1** cytochrom e coxidases ubunit VIa polypeptid e 1 | I | D | Yes | Yes | | | |
| **DUS4L** dihydrouri dinesynth ase 4-like (S. cerevisiae ) | I | D | | | | Benzod iazepines | |
| **FBXW9** F-box and WD repeat domain containing 9 | I | D | Yes | | | | |
| **GAPDH** glyceralde hyde-3-phosphate dehydroge nase | I | D | Yes | | Yes | | |
| **GFPT1** glutamine -fructose-6-phosphate transamin ase 1 | I | D | | | | Antidep ressants | |
| **KIF3C** kinesin family member 3C | D | I | | Yes | | | |
| **MME** membrane metallo-endopepti dase | D | I | | | Yes | | |
| **MYH8** myosin, heavy chain 8, skeletal muscle, perinatal | D | I | | Yes | | | |
| **NAV1** neuron navigator 1 | D | I | Yes | | | Venlafa xine | |
| **NAV3** neuron navigator 3 | D | I | | | | Valproate | |
| **NLGN2** neuroligin 2 | D | I | | Yes | | Valproate | |
| **PCOLCE** procollage n C-endopepti dase enhancer | I | D | Yes | | | | |
| **PEBP1** phosphati dylethanol | I | D | Yes | | | | |
| amine binding protein 1 | | | | | | | |
| **PIWIL4** piwi-like RNA-mediated gene silencing 4 | I | D | | Yes | | | |
| **POLH** polymeras e (DNA directed),e ta | I | D | | | | Carbam azepine | |
| **SCD5** stearoyl-CoA desaturase 5 | | D | Yes | | | | |
| **SCPEP1** serine carboxype ptidase 1 | I | D | | Yes | | | |
| **SIGMAR 1** sigma non-opioid intracellul ar receptor 1 | I | D | | | | | Opioids |

**Table 5. Biomarkers for Active Longevity in Males- Pharmacogenomics for potential stratification and monitoring response to treatment. Biomarker genes that are targets of existing drugs and modulated by them in the same direction as longevity.**

| **Human Gene Symbol/G ene Name** | **Longevity Direction of Change** | **Suicidalit y Direct ion of Change** | **Modulate d by Omega-3** | **Modulate d by Lithium** | **Modulate d by Clozapine** | **Modulate d by Other Psychiatri c Drugs** | **Modulate d by Other Non-Psyc hiatric Drugs** |
|---|---|---|---|---|---|---|---|
| **ANK3** Ankyrin 3 | D | I | Yes | | | | |
| **ASPSCR1** ASPSCR1, UBX Domain Containing Tether For SLC2A4 | I | D | Yes | | | | |
| **CBS** Cystathion ine-Beta-Synthase | I | D | Yes | | | | |
| **CEP250** Centrosom al Protein 250 | D | I | | Yes | | | |
| **COX6A1** Cytochro me C Oxidase-Subunit 6A1Subun it 6A1 | I | D | Yes | Yes | | | |
| **CTSB** Cathepsin B | I | D | Yes | | Yes | | |
| **CYB5A** Cytochro me B5 Type A | I | D | | | Yes | | |
| **CYB5A** Cytochro me B5 Type A | I | D | | | Yes | | |
| **DUS4L** Dihydrouri dine Synthase 4 Like | I | D | | | | Benzodiaz epines | |
| **FABP4** Fatty Acid Binding Protein 4 | I | D | | | | Mood stabilizers | |
| **FBXW9** F-Box And WD Repeat Domain Containing 9 | I | D | Yes | | | | |
| **FUBP1** Far Upstream Element Binding Protein 1 | I | D | Yes | | | | |
| **GAPDH** Glyceralde hy de-3-Phosphate Dehydroge nase | I | D | Yes | | Yes | Benzodiaz epines | |
| **GFPT1** Glutamine -Fructose-6-Phosphate Transamin ase 1 | I | D | | | | Antidepres sants | |
| **KIF3C** Kinesin Family Member 3C | D | I | | Yes | | | |
| **MME** Membrane Metalloen dopeptidas e | D | I | | | Yes | | sacubitril/ valsartan,s acubitril |
| **MRC2** Mannose Receptor C Type 2 | I | D | Yes | | | | |
| **MYH8** Myosin Heavy Chain 8 | D | I | | Yes | | | |
| **NAV1** Neuron Navigator 1 | D | I | Yes | | | SNRIs | |
| **NAV3** Neuron Navigator 3 | D | I | | | | Valproate | |
| **NLGN2** Neuroligin 2 | D | I | | Yes | | Valproate | |
| **NLGN3** Neuroligin 3 | D | I | | | | Valproate | |
| **PCOLCE** Procollage n C-Endopepti dase Enhancer | I | D | Yes | | | | |
| **POLH** DNA Polymeras e Eta | I | D | | | | Mood stabilizers | |
| **PPIF** Peptidylpr oly 1 Isomerase F | I | D | | | Yes | | |
| **RNF141** Ring Finger Protein 141 | D | I | | | Yes | | |
| **SCD5** Stearoyl-CoA Desaturase 5 | I | D | Yes | | | | |
| **SCPEP1** Serine Carboxype ptidase 1 | I | D | | Yes | | | |
| **SIGMAR** 1 Sigma Non-Opioi d Intracellul ar Receptor 1 | I | D | | | | | Opioids |
| **YBX1** Y-Box Binding Protein 1 | I | D | | | Yes | | |

**Table 6. Biomarkers for Active Longevity in Females - Pharmacogenomics for potential stratification and monitoring response to treatment. Biomarker genes that are targets of existing drugs and modulated by them in the same direction as longevity.**

| **Human Gene Symbol/G ene Name** | **Longevity Directiont ion of Change of** | **Suicidalit y Direct Change** | **Modulate d by Omega-3** | **Modu lated by Lithium** | **Modulate d by Clozapine** | **Modulate d by Other Psychiatr ic Drugs** | **Modulate d by Other Non-Psyc hiatric Drugs** |
|---|---|---|---|---|---|---|---|
| **ADAM12** ADAM Metallope ptidase Domain 12 | D | I | Yes | | | | |
| **DUS4L** Dihydrouri dine Synthase 4 Like | I | D | | | | Benzodiaz epines | |
| **EAF1** ELL Associated Factor 1 | I | D | Yes | | | | |
| **GFPT1** Glutamine --Fructose-6-Phosphate Transamin ase 1 | I | D | | | | Antidepre ssants | |
| **H3F3A** H3 Histone Family Member 3A | I | D | Yes | | | Antidepre ssants | |
| **KIF3C** Kinesin Family Member 3C | D | I | | Yes | | | |
| **NAV3** Neuron Navigator 3 | D | I | | | | Valproate | |
| **NLGN1** Neuroligin 1 | D | I | | | Yes | | |
| **PEBP1** Phosphatid ylethanola mine Binding Protein 1 | I | D | Yes | | | | |
| **PIWIL4** Piwi Like RNA-Mediated | I | D | | Yes | | | |
| Gene Silencing 4 | | | | | | | |
| **POLH** DNA Polymeras e Eta | I | D | | | | Mood stabilizers | |
| **RAB14** RAB14, Member | D | I | | | Yes | Antidepre ssants | |
| RAS Oncogene Family | | | | | | | |
| **RHBDF1** Rhomboid 5 Homolog 1 | D | I | | | Yes | | |
| **SSR3** Signal Sequence Receptor Subunit 3 | I | D | | | | Valproate | |
| **TRIP13** Thyroid Hormone Receptor Interactor 13 | I | D | | | | Valproate | |
| **UGDH** UDP-Glucose 6-Dehydroge nase | I | D | | Yes | Yes | | |

### EXAMPLE 4

In this Example, groups of biomarkers were used to identify compounds that produce a similar gene expression signature as active longevity, by matching against the gene expression profiles of thousands of drugs in databases such as the Connectivity Map at Broad Institute/MIT.

Tables 7-9 summarize examples of compounds identified using the gene expression signature of the full lists of biomarkers, the Bonferroni validated sublist, and the top biomarker sublist. An individual can be tested for these panels of biomarkers, and depending how many and which of the markers are changed, can be treated with drugs (pharmaceuticals and/or natural compounds) from among those identified/repurposed. An individual can be treated with a combination of drugs, for example one or more drugs from the universal list, and one or more drugs from the gender list (male or female).

**Table 7. Repurposed Drugs for Active Longevity in Everybody (Universal).**

| compound name | dose | cell | score | gene expression signature |
|---|---|---|---|---|
| isoflupredone | 10 µM | HL60 | 1 | Top Biomarkers |
| Estradiol | 15 µM | HL60 | 1 | Bonferroni Biomarkers |
| ***Genistein*** | 10 µM | MCF7 | 0.997 | Top Biomarkers |
| **Timolol** | 9 µM | MCF7 | 0.98 | Top Biomarkers |
| *alphayohimbine* | 10 µM | MCF7 | 0.96 | Top Biomarkers |
| **rosiglitazone** | 10 µM | HL60 | 0.96 | Bonferroni Biomarkers |
| ***nordihydroguaiaretic acid*** | 1 µM | HL60 | 0.955 | Bonferroni Biomarkers |
| *Monorden* | 100 µM | PC3 | 0.946 | Top Biomarkers |
| ***chenodeoxycholic acid*** | 10 µM | HL60 | 0.946 | Bonferroni Biomarkers |
| **Propranolol** | 14 µM | HL60 | 0.945 | Top Biomarkers |
| Budesonide | 9 uM | HL60 | 0.937 | Bonferroni Biomarkers |
| Dantrolene | 12 uM | MCF7 | 0.936 | Top Biomarkers |
| Spiradoline | 1 µM | MCF7 | 0.936 | Top Biomarkers |
| *dihydroergocristine* | 6 µM | MCF7 | 0.933 | Top Biomarkers |
| Heptaminol | 22 µM | HL60 | 0.932 | Bonferroni Biomarkers |
| SC560 | 10 µM | MCF7 | 0.923 | Top Biomarkers |
| *Vitexin* | 9 µM | HL60 | 0.921 | Top Biomarkers |
| **sirolimus** | 100 µM | MCF7 | 0.919 | Bonferroni Biomarkers |
| beclometasone | 8 µM | PC3 | 0.918 | Top Biomarkers |
| Heptaminol | 22 µM | PC3 | 0.916 | Top Biomarkers |
| Oxybenzone | 18 µM | MCF7 | 0.915 | Top Biomarkers |
| Pirlindole | 12µM | MCF7 | 0.913 | All Biomarkers |
| Moxisylyte | 13 µM | HL60 | 0.908 | Bonferroni Biomarkers |
| *Harmalol* | 15 µM | MCF7 | 0.905 | Top Biomarkers |
| **amitriptyline** | 13 µM | HL60 | 0.904 | Top Biomarkers |
| **6bromoindirubin3'oxime** | 500 µM | MCF7 | 0.901 | Top Biomarkers |

The Universal Longevity Biomarker Signatures matching to the Connectivity Map (Cmap) was used to identify compounds having the same gene expression effects as the longevity biomarkers gene expression signature. A score of 1 means perfect similarity. Sirolimus (rapamycin) is a known-longevity promoting drug, and served as a reassuring positive control. Bold- compounds that are relatively easy to use in the general population. Italic- natural compounds.

**Table 8. Repurposed Drugs for Active Longevity in Males.**

| compound name | dose | cell | score | gene expression signature |
|---|---|---|---|---|
| 01750290000 | 10 µM | PC3 | 1 | Top Biomarkers |
| Estradiol | 10 nM | MCF7 | 1 | All Biomarkers |
| **naftidrofuryl** | 8 µM | PC3 | 0.966 | Top Biomarkers |
| 15 (S)15 methylpro staglandin E2 | 10 µM | MCF7 | 0.949 | All Biomarkers |
| **Cimetidine** | 16 µM | PC3 | 0.935 | Top Biomarkers |
| isocarboxazid | 17 µM | PC3 | 0.924 | All Biomarkers |
| zuclopenthixol | 9 µM | PC3 | 0.923 | Top Biomarkers |
| methocarb amol | 17 µM | MCF7 | 0.918 | All Biomarkers |
| ***pregnenolone*** | 13 µM | PC3 | 0.899 | Top Biomarkers |
| dydrogesterone | 13 µM | PC3 | 0.896 | Top Biomarkers |
| ***calcium folinate*** | 8 µM | MCF7 | 0.888 | All Biomarkers |
| Lidoflazine | 8 µM | PC3 | 0.876 | Top Biomarkers |
| ***docosahexaenoic acid ethyl ester*** | 10 µM | PC3 | 0.873 | Top Biomarkers |
| piperacetazine | 10 µM | PC3 | 0.871 | Top Biomarkers |
| dipyridamole | 8 µM | HL60 | 0.87 | Top Biomarkers |
| mephentermine | 9 µM | PC3 | 0.869 | Top Biomarkers |
| Amantadine | 10 µM | MCF7 | 0.869 | All Biomarkers |
| ipratropium bromide | 10 µM | PC3 | 0.867 | Top Biomarkers |
| fluphenazine | 10 µM | SKMELS | 0.854 | Top Biomarkers |
| ***Kaempferol*** | 14 µM | MCF7 | 0.854 | All Biomarkers |
| ***ursolic acid*** | 9 µM | PC3 | 0.845 | Top Biomarkers |
| Sulpiride | 12 µM | HL60 | 0.844 | All Biomarkers |
| **Memantine** | 19 µM | PC3 | 0.842 | All Biomarkers |
| apomorphine | 6 µM | HL60 | 0.83 | All Biomarkers |
| **Bupropion** | 14 µM | MCF7 | 0.827 | All Biomarkers |
| **Minoxidil** | 19 µM | MCF7 | 0.822 | All Biomarkers |
| Pirlindole | 12 µM | MCF7 | 0.82 | All Biomarkers |

Male Longevity Biomarker Signatures matching to the Connectivity Map (Cmap) were used to identify compounds having the same gene expression effects as the longevity biomarkers gene expression signature. A score of 1 means perfect similarity. Docosahexaenoic acid ethyl ester is a known-longevity promoting compound, and served as a reassuring positive control. Bold-compounds that are relatively easy to use in the general population. Italic- natural compounds.

**Table 9. Repurposed Drugs for Active Longevity in Femals.**

| compound name | dose | cell | score | gene expression signature |
|---|---|---|---|---|
| Carmustine | 100 µM | MCF7 | 1 | All Biomarkers |
| ***asiaticoside*** | 4 µM | HL60 | 0.873 | All Biomarkers |
| ***naringenin*** | 15 µM | PC3 | 0.857 | All Biomarkers |
| oxybenzone | 18 µM | HL60 | 0.856 | All Biomarkers |
| SC560 | 10 µM | MCF7 | 0.847 | All Biomarkers |
| **Verapamil** | 8 µM | PC3 | 0.829 | All Biomarkers |
| ***cyanocobalamin*** | 3 µM | MCF7 | 0.824 | All Biomarkers |
| **Sirolimus** | 100 µM | HL60 | 0.794 | All Biomarkers |
| terconazole | 8 µM | PC3 | 0.79 | All Biomarkers |
| Ketotifen | 9 µM | MCF7 | 0.786 | All Biomarkers |
| **trimipramine** | 10 µM | HL60 | 0.784 | All Biomarkers |

Female Longevity Biomarker Signatures matching to the Connectivity Map (Cmap) were used to identify compounds having the same gene expression effects as the longevity biomarkers gene expression signature. A score of 1 means perfect similarity. Sirolimus (rapamycin) is a known-longevity promoting compound, and served as a reassuring positive control. Bold- compounds that are relatively easy to use in the general population. Italic- natural compounds.

### EXAMPLE 5

In this Example, translational medicine insights were derived.

Previous work demonstrated that ANK3 was increased in expression in the amygdala of a mouse model of mood disorders and stress, and that ANK3 expression was decreased in that model by treatment with the omega-3 fatty acid DHA, similar to the effects of mianserin in worms. A number of other top scoring active longevity genes (Tables 4-6) had evidence of modulation by DHA in the same direction with mianserin, indicating that omega-3 fatty acids can have longevity promoting effects. One of the top biological pathways was linoleic acid metabolism, related to omega-3 fatty acids.

As disclosed herein, a series of biomarkers were identified that changed in opposite directions in longevity vs. in aging and in Alzheimer Disease (Tables 1-3). These biomarkers can serve as targets for early intervention and preventive approaches. COX6A1 (cytochrome c oxidase subunit VIa polypeptide 1, the terminal enzyme of the mitochondrial respiratory chain), and CYB5R3 (cytochrome b5 reductase 3, which functions in desaturation and elongation of fatty acids, in cholesterol biosynthesis, and in drug metabolism), are increased in longevity, and decreased in the blood of Alzheimer Disease individuals. KAT2B (K(lysine) acetyltransferase 2B), which has histone acetyl transferase activity with core histones and nucleosome core particles indicating that this protein plays a direct role in transcriptional regulation), is increased in longevity and decreased in the hippocampus of Alzheimer Diseases individuals.

Another biomarker that is increased in longevity is SRD5A1 (steroid-5-alpha-reductase alpha polypeptide 1). Inhibitors of this enzyme, such as those used in prostate disorders, lead to androgenic blockade, which has been associated with a higher rate of Alzheimer Disease. One of the top biological pathways identified in the Examples was androgen receptor signaling (Table 2).

A number of top biomarkers that were identified have biological roles that are related to the circadian clock. To be able to ascertain all the genes in the dataset that were circadian and to perform estimates for enrichment, a database of all the known genes that fall into three categories: core clock, immediate input or output, and distant input or output, numbering a total of 1468 genes was compiled from the literature. Using an estimate of about 21,000 genes in the human genome, indicates about 7% of genes having some circadian pattern. Out of the 67 top longevity biomarker genes, 11 had circadian evidence (16.4%), indicating a 2-fold enrichment for circadian genes. Circadian clock abnormalities are related to mood disorders and neurodegenerative disorders. Sleep abnormalities have been also implicated in aging.

### EXAMPLE 6

### Longevity v. Suicide

A series of biomarkers have also been identified that appear to change in opposite directed in longevity v. suicide. The genes that have blood evidence in suicide in opposite direction to longevity can be used as blood biomarkers for a biological switch implicated in survival.

### Pharmacogenomics and Therapeutics

A series of biomarkers that seem to be changed in the same direction in longevity vs. in treatments with omega-3 fatty acids, lithium, valproate were identified that can be used to stratify patients to different treatment approaches and to monitor a patient's response (Tables 4-6). COX6A1, SYT1, TROVE2, and NLGN2 were changed in expression by two of these three treatments, indicating they can be core to the mood and longevity mechanisms of these drugs. MYH9, SOD2, COX6A 1, TROVE2, H3F3A, PLA2G6, and PEBP1 can be useful blood pharmacogenomic markers of response to omega-3 fatty acids. Two existing drugs, quinacrine (inhibiting PLA2G6), and sulfinpyrazone (inhibiting ABCC1), used for other indications were identified as targeting top longevity biomarkers, and can be re-purposed for treating acute suicidality.

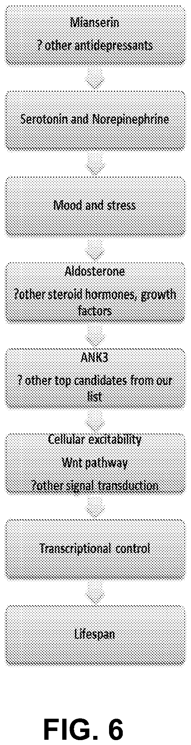

Additionally, Connectivity Map analyses identified compounds that induce gene expression signatures that are the similar to those of the active longevity biomarkers (Table 7-9). Other compounds identified to modulate mood and stress regulated longevity genes, and be used in prolonging lifespan: antidiabetic medications (troglitazone, gliquidone, pioglitazone, rosiglitazone), immunosuppressant/ anti-transplant rejection medications with known longevity effects across species (sirolimus/rapamycin, mycophenolic acid), nootropic (piracetam), and non-drug flavonoid antioxidant/vitamin compounds (quercetin, resveratrol, ergocalciferol/Vitamin D). Known mood modulating drugs identified by the Connectivity Map analyses are: antidepressants (minaprine, amoxapine), antihistamines (homochlorcyclizine), calcium-channel blockers (nifedipine), and female sex hormone-like compounds (diethylstilbestrol, estradiol). Of note, females tend to live longer than males in humans, and estradiol has direct prior experimental evidence of extending lifespan in worms. FIG. 6 is a flow diagram depicting a proposed mechanistic cascade.

### EXAMPLE 7

### Life Switch

A series of biomarkers that changed in the same direction in longevity vs. in treatments with mood stabilizing and anti-suicidal agents such as lithium, clozapine, and omega-3 fatty acids, constituting in essence a "life switch", have been identified. These biomarkers could be used to stratify patients to different treatment approaches, and monitor their response (Tables 4-6).

Using a Connectivity Map, compounds that have similar gene expression signatures to the genes that were changed in opposite direction in suicide and active longevity were identified (Tables 7-9). Additional compounds include flavonoid antioxidants (apigenin, luteolin, acacetin) and vitamins (vitamin K, folic acid), along with resveratrol, estradiol, antidiabetics, and antineoplastics. Moreover, some of the genes in this "life switch" are modulated by omega-3 fatty acids, lithium, and clozapine.

## Claims

1. A method of identifying a modulator of active longevity, the method comprising: providing a C. *elegans* animal; administering a candidate compound to the *C. elegans* animal; and monitoring expression of a C. *elegans* gene chosen from one or more genes in Tables 1, 2 and 3 discovered in human suicide, wherein a change in the expression of C. *elegans* gene in opposite direction to the one in suicide indicates that the candidate compound modulates longevity.

2. A method for determining a biological age score in a subject, the method comprising: determining expression of one or more gene chosen from Tables 1, 2, 3 in a sample obtained from a subject; computing a biological age score from the expression level by computing the Z scores of the expression level of one or more genes, wherein the calculation is gender specific, and identifying the subject as having propensity for active longevity if the biological age score is higher than average population levels for the chronological age of the subject.

3. A method for determining propensity for dying in a subject, the method comprising: determining expression of one or more genes chosen from Tables 1, 2 and 3 in a sample obtained from a subject; identifying the subject as having a propensity for dying by computing a probability of dying if the expression level or slope of change of the one or more gene are higher in the subject compared to the average levels or slope of change in individuals of the same chronological age in the general population.

4. The method of claim 3, wherein the computing a probability of dying is by receiver operating curves area under the curve (ROC AUC), Cox Regressions, and combinations thereof.

5. The method of claim 3, wherein the one or more gene is chosen from Table 1.

6. The method of claim 3, wherein the subject is a male subject and wherein the one or more gene is chosen from Table 2.

7. The method of claim 3, wherein the subject is a female subject and wherein the one or more gene is chosen from Table 3.

8. Compound chosen from one or more compounds in Tables 7, 8 and 9 for use in modulating active longevity in a subject in need thereof, wherein expression of a gene chosen from one or more genes in Tables 1, 2 and 3 discovered in human suicide is monitored, wherein a change in the expression of the gene in the opposite direction of the one in suicide indicates that active longevity is modulated.

9. The use according to claim 8, wherein the compound is chosen from Table 7 and the gene is chosen from Table 1.

10. The use according to claim 8, wherein the subject is a male subject and wherein the compound is chosen from Table 8 and the gene is chosen from Table 2.

11. The use according to claim 8, wherein the subject is a female subject and wherein the compound is chosen from Table 9 and the gene is chosen from Table 3.

## Patentansprüche

1. Verfahren zum Identifizieren eines Modulators von aktiver Langlebigkeit, das Verfahren umfassend: Bereitstellen eines *C. elegans* Tieres; Verabreichen einer Kandidaten-Verbindung an das *C. elegans* Tier; und Überwachen von Expression eines *C. elegans* Gens, ausgewählt aus einem oder mehreren Genen in Tabellen 1, 2 und 3, entdeckt bei humanem Suizid, wobei eine Änderung in der Expression von einem *C. elegans* Gen in entgegengesetzte Richtung als des einen bei Suizid anzeigt, dass die Kandidaten-Verbindung Langlebigkeit moduliert.

2. Verfahren zum Bestimmen eines biologischen Alterswerts in einem Subjekt, das Verfahren umfassend: Bestimmen von Expression eines oder mehrerer Gene, ausgewählt aus Tabellen 1, 2 und 3 in einer Probe, erhalten von einem Subjekt; Errechnen eines biologischen Alterswerts aus dem Expressionsniveau durch Errechnen der Z-Werte des Expressionsniveaus eines oder mehrerer Gene, wobei die Berechnung geschlechtsspezifisch ist, und Identifizieren des Subjekts als eine Neigung für aktive Langlebigkeit zu haben, wenn der biologische Alterswert höher ist als die durchschnittlichen Populationsniveaus für das chronologische Alter des Subjekts.

3. Verfahren zum Bestimmen der Neigung zu Sterben in einem Subjekt, das Verfahren umfassend: Bestimmen der Expression eines oder mehrerer Gene, ausgewählt aus Tabellen 1, 2 und 3 in einer Probe, erhalten von einem Subjekt; Identifizieren des Subjekts als eine Neigung zum Sterben zu haben durch Errechnen einer Wahrscheinlichkeit zu Sterben, wenn das Expressionsniveau oder die Steigung der Veränderung eines oder mehrerer Gene in dem Subjekt verglichen mit den Durchschnittsniveaus oder der Steigung der Veränderung in Individuen desselben chronologischen Alters in der allgemeinen Population höher sind.

4. Verfahren nach Anspruch 3, wobei das Errechnen einer Wahrscheinlichkeit zu Sterben durch Empfänger, anwendend Kurvenbereiche unter der Kurve (ROC AUC), Cox Regression, und Kombination davon, geschieht.

5. Verfahren nach Anspruch 3, wobei das eine oder die mehreren Gene ausgewählt ist aus Tabelle 1.

6. Verfahren nach Anspruch 3, wobei das Subjekt ein männliches Subjekt ist und wobei das eine oder die mehreren Gene ausgewählt ist aus Tabelle 2.

7. Verfahren nach Anspruch 3, wobei das Subjekt ein weibliches Subjekt ist und wobei das eine oder die mehreren Gene ausgewählt ist aus Tabelle 3.

8. Verbindung, ausgewählt aus einer oder mehreren Verbindungen in Tabellen 7, 8 und 9 zur Verwendung für das Modellieren aktiver Langlebigkeit in einem Subjekt, das dessen bedarf, wobei die Expression eines Genes, ausgewählt aus einem oder mehreren Genen in Tabellen 1, 2 und 3, entdeckt bei humanem Suizid, überwacht wird, wobei eine Änderung in der Expression des Gens in entgegengesetzte Richtung als des einen bei Suizid anzeigt, dass aktive Langlebigkeit moduliert wird.

9. Verwendung nach Anspruch 8, wobei die Verbindung ausgewählt ist aus Tabelle 7 und das Gen ausgewählt ist aus Tabelle 1.

10. Verwendung nach Anspruch 8, wobei das Subjekt ein männliches Subjekt ist und wobei die Verbindung ausgewählt ist aus Tabelle 8 und das Gen ausgewählt ist aus Tabelle 2.

11. Verwendung nach Anspruch 8, wobei das Subjekt ein weibliches Subjekt ist und wobei die Verbindung ausgewählt ist aus Tabelle 9 und das Gen ausgewählt ist aus Tabelle 3.

## Revendications

1. Procédé d'identification d'un modulateur de longévité active, le procédé comprenant: la fourniture d'un animal *C. elegans;* l'administration d'un composé candidat à l'animal *C. elegans,* et la surveillance de l'expression d'un gène de *C. elegans* choisi parmi un ou plusieurs gènes dans les tableaux 1, 2 et 3 découverts dans le suicide humain, dans lequel un changement dans l'expression du gène de *C. elegans* dans le sens opposé à celui du suicide indique que le composé candidat module la longévité.

2. Procédé pour déterminer un score d'âge biologique chez un sujet, le procédé comprenant: la détermination de l'expression d'un ou plusieurs gènes choisis à partir des tableaux 1, 2, 3 dans un échantillon obtenu à partir d'un sujet; le calcul d'un score d'âge biologique à partir du niveau d'expression en calculant les scores Z du niveau d'expression d'un ou plusieurs gènes, dans lequel le calcul est spécifique du sexe, et l'identification du sujet comme ayant une propension à la longévité active si le score d'âge biologique est supérieur à des niveaux de population moyens pour l'âge chronologique du sujet.

3. Procédé pour déterminer la propension à mourir chez un sujet, le procédé comprenant: la détermination de l'expression d'un ou plusieurs gènes choisis à partir des tableaux 1, 2 et 3 dans un échantillon obtenu à partir d'un sujet; l'identification du sujet comme ayant une propension à mourir en calculant une probabilité de mourir si le niveau d'expression ou la pente de changement des un ou plusieurs gènes sont plus élevés chez le sujet par rapport aux niveaux moyens ou à la pente de changement chez des individus du même âge chronologique dans la population générale.

4. Procédé selon la revendication 3, dans lequel le calcul d'une probabilité de mourir est par l'aire sous la courbe de courbes de fonctionnement du récepteur (ROC AUC), des régressions de Cox et leurs combinaisons.

5. Procédé selon la revendication 3, dans lequel les un ou plusieurs gènes sont choisis à partir du tableau 1.

6. Procédé selon la revendication 3, dans lequel le sujet est un sujet mâle et dans lequel les un ou plusieurs gènes sont choisis à partir du tableau 2.

7. Procédé de la revendication 3, dans lequelle le sujet est un sujet femelle et dans lequel les un ou plusieurs gènes sont choisis à partir du tableau 3.

8. Composé choisi parmi un ou plusieurs composés dans les tableaux 7, 8 et 9 pour une utilisation dans la modulation de la longévité active chez un sujet qui en a besoin, dans lequel l'expression d'un gène choisi parmi un ou plusieurs gènes dans les tableaux 1, 2 et 3 découverts dans le suicide humain est surveillée, dans lequel un changement dans l'expression du gène dans le sens opposé à celui du suicide indique que la longévité active est modulée.

9. Utilisation selon la revendication 8, dans laquelle le composé est choisi à partir du tableau 7 et le gène est choisi à partir du tableau 1.

10. Utilisation selon la revendication 8, dans laquelle le sujet est un sujet mâle et dans laquelle le composé est choisi à partir du tableau 8 et le gène est choisi à partir du tableau 2.

11. Utilisation selon la revendication 8, dans laquelle le sujet est un sujet femelle et dans laquelle le composé est choisi à partir du tableau 9 et le gène est choisi à partir du tableau 3.
